# EUROPEAN PATENT SPECIFICATION

(11) **EP 2 899 266 B1**
(45) Date of publication and mention of the grant of the patent: **07.03.2018**
(21) Application number: 14152494.2
(22) Date of filing: 24.01.2014
(51) Int. Cl.: C12N 5/077

(54) **IMPROVED DIFFERENTIATION OF MESENCHYMAL STEM CELLS INTO OSTEOBLASTS**
VERBESSERTE DIFFERENZIERUNG MESENCHYMALER STAMMZELLEN ZU OSTEOBLASTEN
DIFFÉRENTIATION AMÉLIORÉE DE CELLULES SOUCHES MÉSENCHYMATEUSES EN OSTÉOBLASTES

(43) Date of publication of application: 29.07.2015
(73) Proprietor: Fresenius Medical Care Deutschland GmbH, 61352 Bad Homburg (DE)
(72) Inventor: Munoz Castaneda, Juan Rafael, 14004 Cordoba (ES); Diaz Tocados, Juan Miguel, 14004 Cordoba (ES); Herencia Bellido, Carmen, 14004 Cordoba (ES); Rodriguez Ortiz, Maria Encarnacion, 14004 Cordoba (ES); Montes de Oca Gonzalez, Addy Rosa, 14004 Cordoba (ES); Martinez-Moreno, Julio Manuel, 14004 Cordoba (ES); Almaden Pena, Yolanda, 14004 Cordoba (ES); Rodriguez Portillo, Mariano, 14004 Cordoba (ES); Aljama Garcia, Pedro, 14004 Cordoba (ES); Gundlach, Kristina, 55126 Mainz (DE); Mirjam, Peter, 60389 Frankfurt am Main (DE); Büchel, Janine, 61350 Bad Homburg (DE); Steppan, Sonja, 63263 Neu-Isenburg (DE); Passlick-Deetjen, Jutta, 77960 Seelbach (DE)
(74) Representative: Weiß, Stefan

(56) References cited:
- WO-A1-2013/013648
- WO-A2-2005/039662
- US-A1- 2011 212 523
- HUSSAIN AHMED ET AL: "Magnesium calcium phosphate as a novel component enhances mechanical/physical properties of gelatin scaffold and osteogenic differentiation of bone marrow mesenchymal stem cells.", TISSUE ENGINEERING. PART A APR 2012, vol. 18, no. 7-8, April 2012 (2012-04), pages 768-774, XP055112850, ISSN: 1937-335X
- ABED ELIE ET AL: "Importance of melastatin-like transient receptor potential 7 and magnesium in the stimulation of osteoblast proliferation and migration by platelet-derived growth factor", AMERICAN JOURNAL OF PHYSIOLOGY - CELL PHYSIOLOGY, vol. 297, no. 2, August 2009 (2009-08), pages C360-C368, XP055112851, ISSN: 0363-6143
- SHIMAYA M ET AL: "Magnesium enhances adherence and cartilage formation of synovial mesenchymal stem cells through integrins", OSTEOARTHRITIS AND CARTILAGE, BAILLIERE TINDALL, LONDON, GB, vol. 18, no. 10, 1 October 2010 (2010-10-01), pages 1300-1309, XP027294616, ISSN: 1063-4584 [retrieved on 2010-07-13]

## Description

The present invention relates to a method for the generation of osteoblasts and the formation of bone. In particular the invention relates to the ability of magnesium ions to enhance differentiation of mesenchymal stem cells into osteoblasts and further the growth thereof. The invention is based on the finding that unphysiologically high extracellular concentrations of magnesium have a stronger effect on differentiation and growth compared to physiological concentrations of magnesium.

Magnesium is the fourth most abundant cation in the human body, and the first one among divalent cations. Magnesium is an essential electrolyte involving several enzymatic reactions, as catalyst or enzymatic cofactor, basic cellular process, such as energy obtaining and protein and DNA synthesis, and mineral balance together with other ions such calcium, sodium and potassium. Magnesium is mainly stored in bone tissue (approximately 55% of the total magnesium in the body) and is responsible for the homeostasis maintenance and bone functionality.

Magnesium deficiencies had been associated to pathologic process as osteoporosis, hypertension, arrhythmia, heart stroke, diabetes mellitus or (pre-) eclampsia.

In respect to bone, several studies have reported that low magnesium levels (hypomagnesemia) are related to bone dysfunction. Thus, it had been extensively reported that osteoporotic patients show low serum magnesium levels. Further magnesium deficiency and alterations in the mineral metabolism lead to osteodystrophy, which frequently entails bone mineral density or osteopenia.

Magnesium supplementation had been related to decreasing of vascular calcification and cardiovascular risk. However, the role of magnesium in bone remains controversial. It had been observed that magnesium supplementation in the diet decreases parathyroid hormone (PTH) levels leading to a decrease in bone resorption.

Mesenchymal stem cells (MSC) are osteoblast progenitor cells, with high potential to be differentiated into osteoblast under osteogenic stimulus, containing dexamethasone, ascorbic acid and glycerolphosphate.

EP1684815 describes that unphysiologically high concentrations of extracellular magnesium stimulate growth and regeneration of chondrons.

EP1259196 describes a bone graft substitute. Incorporation of a material comprising magnesium salt is mentioned.

It is an object of the invention to provide an effective method of differentiating osteoblasts from MSC. This MSC could be derived of bone marrow, adipose tissue, umbilical cord blood, or adult muscle. Preferably the MSC are adult stem cells. In the context of this invention "adult stem cells" means the stem cell is derived from fully developed post-natal tissue or blood, in contrast to being derived from embryonic or fetal cells.

The objects of the invention are met by claims 1 - 5.

It has been found that the differentiation of MSC into osteoblasts is enhanced when the differentiation medium comprises unphysiologically high extracellular concentrations of magnesium. This effect can be utilized by supplementation of a differentiation medium for MSC by unphysiologically high extracellular concentrations of magnesium and a method of differentiating MSC in such a medium in vitro. Resulting differentiated osteoblasts may be used for transplantation in regenerative medicine. The effect may further be utilized by administration of a composition comprising MSC suspended in a differentiation medium with an unphysiologically high concentration of magnesium. According to the invention magnesium may also be utilized in a pharmaceutical composition or in a bone scaffold to enhance the differentiation and growth of osteoblasts by administration of such medicinal compositions or transplantation of such medical devices. In such a case the composition of the scaffold material comprising the magnesium results in a microenvironment with unphysiologically high concentrations of magnesium and thereby stimulating resident MSC to an enhanced differentiation and bone formation. Said scaffolds and pharmaceutical compositions may therefore be advantageously used for the treatment of bone disease such as bone injury, bone trauma, osteoporosis, osteonecrosis and dental bone loss.
Figure 1 shows an increase of alkaline phosphatase activity which indicates proliferation of osteoblasts in vitro at different magnesium concentrations.
Figure 2 shows bone matrix mineralization indicated by Alizarin Red Staining at different magnesium concentrations.
Figure 3 shows the expression of osteogenic genes during osteogenesis at different magnesium concentrations indicating the level of osteogenesis.
Figure 4 shows the level of FGF23 secretion at different magnesium concentrations indicating the maturation of osteoblasts.
Figure 5 shows the increase of cellular proliferation of MSC during osteogenesis indicated by the levels of Cyclin D1 and PCNA.

The term magnesium as relevant for this application is defined by uncharged magnesium which may be protein bound, complexed magnesium as well as predominantly in ionic form. Typically extracellular magnesium is found in the human body in its ionic form. For the purpose of this invention magnesium may be provided in form of one of its salts, oxides or hydroxids. Preferably magnesium is provided in form of solubilized magnesium chloride or magnesium sulfate.

The term "unphysiologically high extracellular concentration of magnesium" as relevant for this invention means that the concentration of magnesium in culture is above the level normally present in the human body. In the human body a concentration between 0.6 mmol/l and 1 mmol/l of serum magnesium can be found physiologically. An unphysiologically high concentration means that the concentration of magnesium in the extracellular compartment is above said physiological concentration.

Mesenchymal stem cells are pluripotent progenitor cells with a high potential to proliferate and to differentiate into different cell types including osteoblasts, chondrocytes and adipocytes. To proliferate in vitro they need to be suspended in a culture medium providing sufficient nutrients. Several suitable culture media are known to the skilled person. One preferred culture medium is α-MEM. The culture medium may optionally be supplemented with fetal calf serum.

To advance differentiation of MSC into differentiated cells a stimulus may be added to the culture medium which thereby becomes a differentiation medium. To stimulate differentiation into osteoblast the addition of one or more substances selected from the group of ascorbic acid, β-glycerol phosphate and dexamethasone may be provided in the culture medium.

It has been found that adding unphysiologically high extracellular concentrations of magnesium increases both speed differentiation and the number of MSC which undergo differentiation. This results in a higher number of osteoblasts in vitro. The same is envisioned to be the case in vivo resulting in an enhanced bone formation and mineralization.

Due to in vitro experiments it has been found that magnesium concentrations of more than 1 mmol/l can enhance MSC differentiation into osteoblasts. A preferred magnesium concentration is in the range of 1.2 - 100 mmol/l, a specifically preferred range is 1.5 - 50 mmol/l and the most preferred range is 1.8 - 4.5 mmol/l.

Osteoblasts resulting from a differentiation method as described above may be used in regenerative medicine wherever an increased formation of bone is advised. This may be the case in various kinds of bone diseases including bone injury, bone trauma, osteoporosis, osteonecrosis and dental bone loss. To treat such bone diseases differentiated osteoblasts may be transplanted. Alternatively mesenchymal stem cells in a differentiation medium as described above may be administered to the patient.

It is also an advantageous aspect of the invention to establish an appropriate microenvironment in the patient's body which allows enhanced growth and differentiation of mesenchymal stem cells and/or osteoblasts. This can be achieved by either supplementing the patient with a pharmaceutical composition which has a high concentration of magnesium, preferably above 1 mmol/l. Such a pharmaceutical composition is preferably administered locally to establish the microenvironment. It may also be administered systemically, for example orally. In that case care has to be taken that supplementation of magnesium does not result in severe hypermagnesemia.

In bone injury or trauma scaffolds are often used to rebuild or regenerate bone. Such scaffolds are typically made from a ceramic or polymeric material. Preferred materials for scaffolds are based on hydroxyapatite. Growth of osteoblasts and differentiation of MSC into osteoblasts advance the formation of new bone material. Both can be furthered by providing a microenvironment comprising magnesium at an unphysiologically high concentration. Such concentrations may be established by providing scaffold materials which have magnesium incorporated therein in a form in which the magnesium is made bioavailable and is released into the microenvironment over time. The magnesium may be provided in the material of the scaffold as such or preferably it is provided as a coating of the scaffold. Various forms of provision of materials or coatings in which a mineral with enhanced bioavailability is provided are known to the skilled person.

### Examples

### Isolation of Rat Bone Marrow MSCs

Animals were anesthetized with pentobarbital sodium (50 mg/kg). Tibias and femurs of ten male Wistar rats cut at the epiphyses were perfused with alpha minimal essential medium (αMEM) (Sigma-Aldrich, St. Louis, MO) supplemented with 15% fetal bovine serum (FBS) (Lonza Walkersville, Inc., USA), 1% ultraglutamine (Lonza Walkersville, Inc., USA), 100 U/mL penicillin and 100 µg/mL streptomycin. Single-cell suspension was generated by filtration trough 70µm cell strainer (BD Falcon, San Jose, California, USA). Following centrifugation and washing with αMEM medium, bone marrow stem cells were harvested in 25 cm² flasks (NUNC, A/S, Roskilde, De) with αMEM containing 15% FBS, 1% ultraglutamine, 100 U/mL penicillin, 100 mg/mL streptomycin and 1 ng/mL of basic fibroblast growth factor (bFGF) (PeproTech EC Ltd, London, UK) and then cultured in a humidified atmosphere of 5% CO₂ at 37°C. Fresh α-MEM medium containing 10% FBS, 1% ultraglutamine (Lonza Walkersville, Inc., USA), 100 U/mL penicillin, 100 µg/mL streptomycin and 1 ng/mL bFGF was added after 24 h and changed every 3 days. Once 85-90% confluent, cells were collected using Trypsin-EDTA (Lonza Walkersville, Inc., USA) and seeded in 6-well plates at 13000 cells/cm² in αMEM containing 10% FBS, 1% ultraglutamine (Lonza Walkersville, Inc., USA), 100 U/mL penicillin, 100 µg/mL streptomycin and 1 ng/mL bFGF. Treatments were started as described below when cells reached confluence.

### MSC differentiation into osteoblasts

MSC were cultured for 21 days in αMEM containing 1µM dexamethasone (Sigma-Aldrich, St. Louis, MO), 10 mM β-glycerol phosphate (Sigma-Aldrich, St. Louis, MO) and 0.2 mM ascorbic acid (Química Farmacéutica BAYER, Barcelona, Spain) and α-MEM with 10 % FBS. In addition, different MgCl₂ (Carlo Erba Reagenti SpA, Milano, IT) concentrations (1.2 and 1.8 mM) were added within osteogenic stimulus.

### Quantitative real time RT-PCR measurement of gene expression

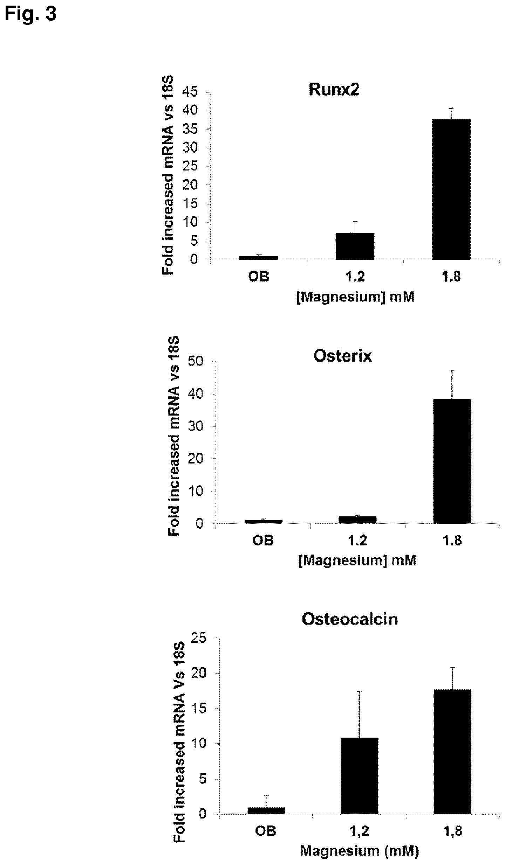

Total RNA was extracted with TRIzol reagent (Sigma-Aldrich, Co, St Louis, Mo) and was quantified by spectrophotometry (ND-1000, Nanodrop Technologies, Wilmington, DE). cDNA was synthesized from 1µg of total RNA with a first strand cDNA synthesis kit (Qiagen; Hilden, Germany) in the presence of random hexamers in a final volume of 20 µl at 25°C for 10 min, followed by 42°C for 15 min and 95°C for 3 min. RT-PCR SYBR Green kit (Qiagen; Hilden, Germany) was used to quantify mRNA expression levels. Runx2, Osterix and Osteocalcin were synthesized with Oligo program and PPARγ was purchased from Qiagen (Qiagen; Hilden, Germany). The expressions of these genes were evaluated by quantitative real time RT-PCR (Light cycler, Roche Diagnostics, Basel, Suiza) using ribosomal 18S RNA as housekeeping gene. The results of the gene expression experiments are shown in Figure 3. The amount of osteogenic genes expressed increases with the concentration of magnesium in the differentiation medium. This indicates that higher concentrations of magnesium enhance differentiation of MSC into osteoblasts.

### Protein extraction and western blot analysis

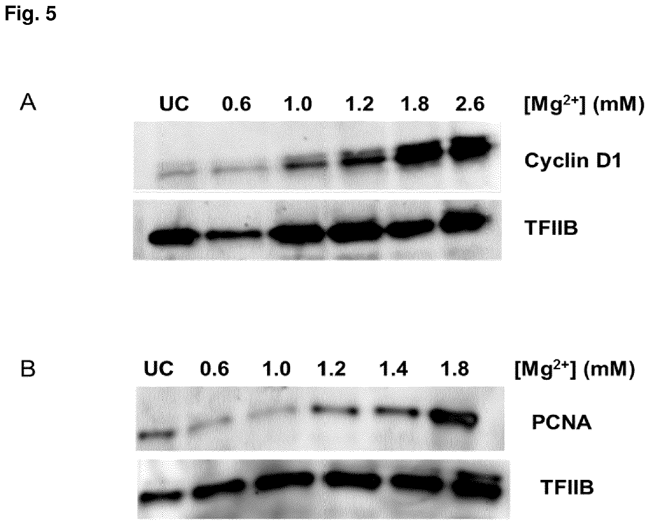

Cytosolic protein was isolated from MSC in a lysis buffer containing 10 mM Hepes, 10 mM KCl, 0.1 mM EDTA, 0.1 mM EGTA, 1 mM DTT, 0.5 mM PMSF, 70 µg/mL Protease Inhibitor Cocktail (Sigma-Aldrich, Co, St Louis, Mo), 0.5% Igepal CA-630 (Sigma-Aldrich, Co, St Louis, Mo), pH 7.9. Suspension was centrifuged and supernatant (cytosolic extract) was stored. Nuclear extracts were obtained by incubating the pellet obtained from the cytosolic extract in a lysis buffer containing 20 mM HEPES, 0.4 mM NaCl, 1 mM EDTA, 1 mM EGTA, 1 mM DTT, 1 mM PMSF, 46 µg/mL Protease Inhibitor Cocktail, pH 7.9. Protein concentration was determined by Bradford assay (Bio-Rad Laboratories GmbH, Munich, Germany). To determine specific proteins content, 20 µg of nuclear cell lysates were analyzed by immunoblotting using antibodies for PCNA (Santa Cruz Biotechnology INC, Dallas, TX) at 1:100 and Cyclin D1 (Cell Signaling Technology Inc., Danvers, MA) at 1:500 as primary antibodies, and horseradish peroxidase-conjugated goat anti mouse (Santa Cruz Biotchnology Inc., Dallas, TX) at 1:10000 and goat anti rabbit (Santa Cruz Biotechnology Inc, Dallas, TX) at 1:10000 as secondary antibodies. Transcription factor II B (TFIIB) (Cell Signaling Technology Inc., Dallas, TX) was used as loading control. The results shown in Fig. 5 and indicate that the addition of magnesium increases cellular proliferation during osteogenesis of rat MSC.

### Alkaline Phosphatase (ALP) Activity

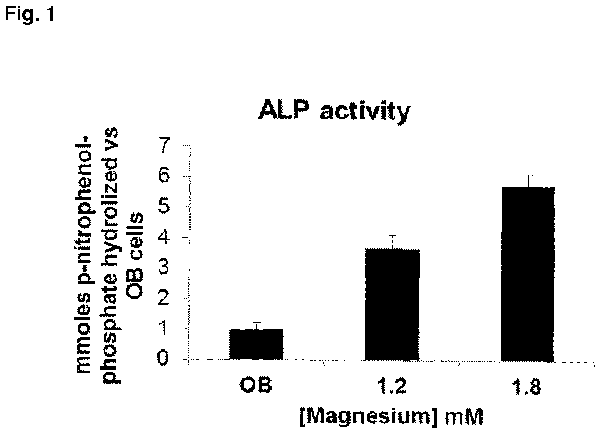

2 µg of protein were used to measure alkaline phosphatase specific activity. p-nitrophenolphosphate (Sigma-Aldrich) 5 mM was used as a substrate. Cell lysates were incubated in 2 mM p-nitrophenol phosphate for 30 minutes at 37°C. The reaction was stopped by adding 3 M NaOH, and the product was quantified at 405 nm. ALP activity was expressed as hydrolyzed mmoles of p-nitrophenol phosphate per minute and per microgram of protein. Results are expressed as comparison with osteoblasts cells in Figure 1. The amount of alkaline phosphatase activity increases with the concentration of magnesium in the differentiation medium. This indicates a higher osteoblast activity.

### Alizarin red S staining

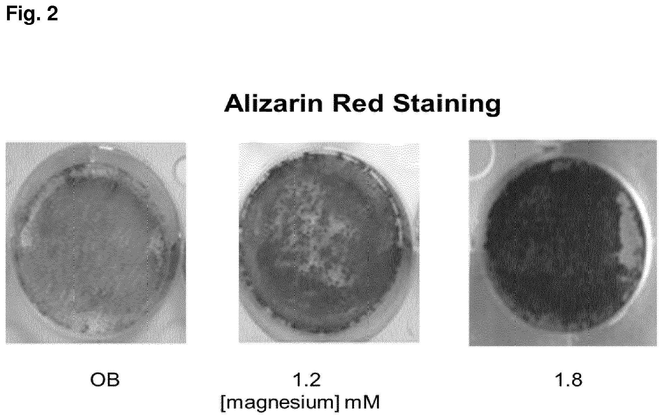

Matrix mineralization was detected by alizarin red S staining (Sigma-Aldrich, St. Louis, MO) 40 mM. Cells were washed twice with PBS, fixed with 2% paraformaldehyde 1% sucrose for 15 minutes and washed with PBS 3 times. Following cells were stained with alizarin red S 40 mM pH 4.1 for 20 minutes, and washed 4 times for 5 minutes with water pH 7 with shaking. After, water was removed and samples were dried at room temperature. The results are shown in Figure 2. Increased concentrations of magnesium result in an improved mineralization of osteoblasts during osteogenesis.

### FGF23 releasing quantification

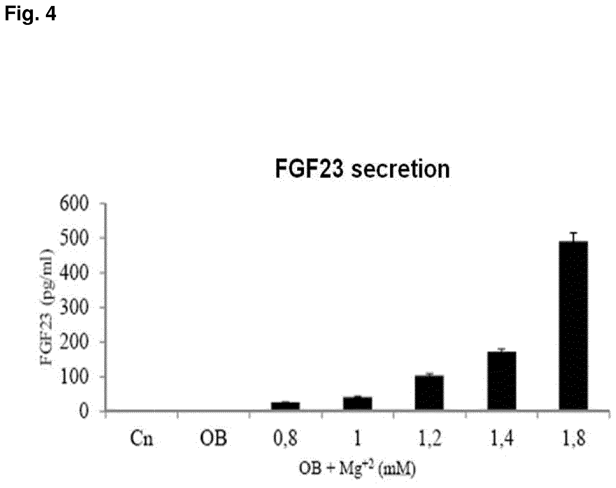

Supernatant from the culture were collected and full length of FGF23 content was determined by specific ELISA kit (Kainos Laboratories, Tokyo, Japan). The result is shown in Figure 4. The amount of FGF23 secretion indicates the maturation of osteoblasts. Unphysiologically high concentrations of magnesium result in an increased number of mature osteoblasts compared to a physiological concentration of magnesium.

## Claims

1. Method for the generation of osteoblasts comprising the step of cultivation of mesenchymal stem cells in a differentiation medium comprising an unphysiologically high extracellular concentration of magnesium wherein the concentration of magnesium is 1.2 mmol/l - 100 mmol/l.

2. Method according to claim 1 **characterized in that** said magnesium is provided in form of a solution of a magnesium salt, preferably magnesium chloride or magnesium sulfate.

3. Method according any one of the preceding claims **characterized in that** the concentration of magnesium is 1.5 mmol/l - 50 mmol/l, most preferably 1.8 - 4.5 mmol.

4. Method according any one of the preceding claims **characterized in that** said differentiation medium comprises one or more differentiation factors selected from the group of dexamethasone, β-glycerol phosphate and ascorbic acid.

5. Method according any one of the preceding claims **characterized in that** said differentiation medium comprises α-MEM.

## Patentansprüche

1. Verfahren zur Erzeugung von Osteoblasten, umfassend den Schritt des Kultivierens von mesenchymalen Stammzellen in einem Differenzierungsmedium, das eine unphysiologisch hohe extrazelluläre Konzentration an Magnesium umfasst, wobei die Magnesiumkonzentration 1,2 mmol/l - 100 mmol/l beträgt.

2. Verfahren nach Anspruch 1, **dadurch gekennzeichnet, dass** das Magnesium in Form einer Lösung eines Magnesiumsalzes, vorzugsweise Magnesiumchlorid oder Magnesiumsulfat, bereitgestellt wird.

3. Verfahren nach einem der vorhergehenden Ansprüche, **dadurch gekennzeichnet, dass** die Magnesiumkonzentration 1,5 mmol/l - 50 mmol/l, am stärksten bevorzugt 1,8 mmol/l - 4,5 mmol/l, beträgt.

4. Verfahren nach einem der vorhergehenden Ansprüche, **dadurch gekennzeichnet, dass** das Differenzierungsmedium einen oder mehrere Differenzierungsfaktoren umfasst, die aus der Gruppe bestehend aus Dexamethason, β-Glycerolphosphat und Ascorbinsäure ausgewählt werden.

5. Verfahren nach einem der vorhergehenden Ansprüche, **dadurch gekennzeichnet, dass** das Differenzierungsmedium α-MEM umfasst.

## Revendications

1. Procédé de génération d'ostéoblastes, comprenant l'étape de culture de cellules souches mésenchymateuses dans un milieu de différentiation comprenant une concentration extracellulaire non physiologiquement élevée de magnésium, la concentration de magnésium étant de 1,2 mmol/l - 100 mmol/l.

2. Procédé selon la revendication 1, **caractérisée en ce que** ledit magnésium est fourni sous forme d'une solution de sel de magnésium, de préférence de chlorure de magnésium ou de sulfate de magnésium.

3. Procédé selon l'une quelconque des revendications précédentes, **caractérisé en ce que** la concentration de magnésium est de 1,5 mmol/l - 50 mmol/l, plus préférentiellement de 1,8 - 4,5 mmol.

4. Procédé selon l'une quelconque des revendications précédentes, **caractérisé en ce que** ledit milieu de différentiation comprend plusieurs facteurs de différenciation sélectionnés dans le groupe composé du déxaméthasone, β-glycérol phosphate et de l'acide ascorbique.

5. Procédé selon l'une quelconque des revendications précédentes, **caractérisé en ce que** ledit procédé de différenciation comprend du α-MEM.
